(19) Europäisches Patentamt

European Patent Office

Office européen des brevets

(11) Veröffentlichungsnummer: **0 355 175**
**A1**

(12) **EUROPÄISCHE PATENTANMELDUNG**

(21) Anmeldenummer: 88113360.7

(22) Anmeldetag: 17.08.88

(51) Int. Cl.⁴: **A61B 17/22 , A61B 8/14 , G01S 15/89**

(43) Veröffentlichungstag der Anmeldung:
28.02.90 Patentblatt 90/09

(84) Benannte Vertragsstaaten:
**DE FR GB NL**

(71) Anmelder: **Siemens Aktiengesellschaft Wittelsbacherplatz 2 D-8000 München 2(DE)**

(72) Erfinder: **Hassler, Dietrich, Dipl.-Ing. Flugweg 3 D-8525 Uttenreuth(DE)**
Erfinder: **Schmidt, Erhard Heuwaagstrasse 20 D-8520 Erlangen(DE)**

(54) **Einrichtung zum berührungslosen Zertrümmern von Konkrementen im Körper eines Lebewesens.**

(57) Die Erfindung betrifft eine Einrichtung zum berührungslosen Zertrümmern von Konkrementen (14) im Körper (4) eines Lebewesens, welcher eine Stoßwellenquelle (2, 10) zur Erzeugung von in einer Fokuszone (F) zusammenlaufenden Stoßwellen und eine Ultraschall-Ortungseinrichtung mit einem B-Scan-Applikator (15) aufweist, mittels dessen zumindest die Fokuszone (F) abtastbar ist. Der B-Scan-Applikator (15) enthält mehrere Ultraschall-Transducer (17a, 17b, 17c), mittels derer wenigstens quasigleichzeitig eine der Anzahl der Ultraschall-Transducer (17a, 17b, 17c) entsprechende Anzahl zumindest näherungsweise parallel zueinander verlaufender Schichten abtastbar ist, deren im Bereich der Fokuszone (F) befindliche Abschnitte einander direkt benachbart sind. Es besteht so die Möglichkeit, Verlagerungen eines zu zertrümmernden Konkrementes (14) quer zur Richtung der Schichten während der Behandlung zu beobachten.

FIG 1

EP 0 355 175 A1

## Einrichtung zum berührungslosen Zertrümmern von Konkrementen im Körper eines Lebewesens

Die Erfindung betrifft eine Einrichtung zum berührungslosen Zertrümmern von Konkrementen im Körper eines Lebewesens, welche eine Stoßwellenquelle zur Erzeugung von in einer Fokuszone zusammenlaufenden Stoßwellen, eine Ultraschall-Ortungseinrichtung mit einem B-Scan-Applikator, mittels dessen zumindest die Fokuszone abtastbar ist, und Mittel zur akustischen Koppelung der Stoßwellenquelle und des B-Scan-Applikators mit dem Körper des Lebewesens aufweist.

Bei der Behandlung von Steinleiden mittels derartiger Einrichtungen wird so verfahren, daß die Einrichtung und der Körper des zu behandelnden Lebewesens mit Hilfe der Ultraschall-Ortungseinrichtung so relativ zueinander ausgerichtet werden, daß sich das zu zertrümmernde Konkrement, z.B. ein Nierenstein, im Fokus der Stoßwellen befindet. In den Körper des Lebewesens werden dann fokussierte Stoßwellen eingeleitet, unter deren Wirkung das Konkrement in Fragmente zerfällt, die auf natürlichem Wege abgehen können.

Eine Einrichtung der eingangs genannten Art ist in der EP-A-0 148 653 beschrieben. Bei dieser Einrichtung wird mittels des B-Scan-Applikators eine die akustische Achse der Stoßwellenquelle enthaltende Schicht des Körpers des zu behandelnden Lebewesens abgetastet, um ein zu zertrümmerndes Konkrement lokalisieren zu können. Wandert während der Behandlung das Konkrement, z.B. infolge der Atemtätigkeit des zu behandelnden Lebewesens oder infolge der Wirkung der Stoßwellen, aus der mittels des B-Scan-Applikators abtastbaren Körperschicht heraus, entzieht es sich der kontinuierlichen Darstellung. Dies ist nachteilig und auch dann nicht ganz vermeidbar, wenn Maßnahmen getroffen sind, die es gestatten, den B-Scan-Applikator so auszu richten, daß die Mittelebene der abtastbaren Schicht mit der im Zusammenhang mit der Atemtätigkeit des Lebewesens auftretenden Haupt-Verschieberichtung des Konkrementes zusammenfällt. Sollen die Einrichtung und der Körper des Lebewesens nach einem Auswandern des Konkrementes relativ zueinander wieder so ausgerichtet werden, daß das Konkrement in der mittels des B-Scan-Applikators abtastbaren Schicht liegt, so fehlt die Information darüber, auf welcher Seite der Schicht das Konkrement liegt. Es muß daher "blind" gesucht werden. Dies kostet Zeit und ist mühsam.

Es wurde daher bereits vorgeschlagen, einen zweiten B-Scan-Applikator vorzusehen, mittels dessen eine zweite, ebenfalls die akustische Achse der Stoßwellenquelle enthaltende Körperschicht abtastbar ist, die mit der ersten Körperschicht einen Winkel einschließt. Es werden so zwar zusätzliche Informationen erhalten, die jedoch nur dann hilfreich sind, wenn sich das Konkrement nur geringfügig im Bereich der Fokuszone verlagert.

Der Erfindung liegt die Aufgabe zugrunde, eine Einrichtung der eingangs genannten Art so auszubilden, daß Verlagerungen des Konkrementes in einer solchen Weise erkannt werden können, daß die Lage der Einrichtung und des Körpers des zu behandelnden Lebewesens relativ zueinander in einfacher Weise und zeitsparend korrigiert werden kann.

Nach der Erfindung wird diese Aufgabe dadurch gelöst, daß der B-Scan-Applikator mehrere Ultraschall-Transducer enthält, mittels derer wenigstens quasi-gleichzeitig eine der Anzahl der Ultraschall-Transducer entsprechende Anzahl zumindest näherungsweise parallel zueinander verlaufender Schichten abtastbar ist, deren im Bereich der Fokuszone befindliche Abschnitte einander direkt benachbart sind. Es können dann selbst größere Verlagerungen des Konkrementes sicher erkannt werden, da das Konkrement bei Verlassen einer der Schichten in die jeweils direkt benachbarte Schicht eintritt und somit kontinuierlich abgebildet wird, wobei erkannt werden kann, in welcher Richtung sich das Konkrement verlagert. Die Lage der Einrichtung und des Körpers des zu behandelnden Lebewesens relativ zueinander kann also sofort gezielt korrigiert werden, indem z.B. die Einrichtung so gekippt wird, daß sich das zu zertrümmernde Konkrement wieder in der Fokuszone bzw. der mittleren B-Scan-Ebene befindet. Vorzugsweise verlaufen die Schichten etwa parallel zur akustischen Achse der Stoßwellenquelle, auf der die Fokuszone der Stoßwellen liegt.

Gemäß einer bevorzugten Ausführungsform der Erfindung besitzt die Stoßwellenquelle eine akustische Achse, die durch die Fokuszone verläuft, und enthält der B-Scan-Applikator eine ungerade Anzahl von Ultraschall-Transducern, die derart angeordnet sind, daß die mittlere der abtastbaren Schichten die akustische Achse der Stoßwellenquelle enthält. Liegt das zu zertrümmernde Konkrement zunächst in der mittleren Schicht, so kann ohne weiteres beobachtet werden, nach welcher Seite das Konkrement aus dieser Schicht herauswandert. Vorzugsweise sind die Ultraschall-Transducer relativ zueinander derart angeordnet, daß die Mittelebenen der abtastbaren Schichten parallel zueinander verlaufen, so daß auch dann, wenn die Stoßwellenquelle einen veränderbaren Fokusabstand aufweist, stets gewährleistet ist, daß die im Bereich der Fokuszone befindlichen Abschnitte der Schichten einander direkt benachbart sind. Insbesondere im Zusammenhang mit Stoßwellenquellen mit festem Fokusabstand kann aber auch vorgesehen sein, daß die Ultraschall-Transducer relativ zueinander derart angeordnet sind, daß die Mittelebenen der abtastbaren Schichten in Richtung auf die Fokuszone divergieren. Dabei ist die Divergenz der Mittelebene in Abhängigkeit von der Lage der Fokuszone derart gewählt, daß die im Bereich der Fokuszone befindlichen Abschnitte der Schichten einander direkt benachbart sind. Im Gegensatz zu einer Anordnung der Ultraschall-Transducer, bei der die Mittelebenen der abtastbaren Schichten parallel zueinander verlaufen und die Ultraschall-Transducer demnach seitlich gegeneinander versetzt angeordnet sein müssen, ist dieser Versatz bei in Richtung auf die Fokuszone divergierenden Mittelebenen der abtastbaren Schicht nicht oder nur in geringem Maße erforderlich. Es genügt eine gegenseitige Verkippung, so daß eine raumsparende Bauweise des B-Scan-Applikators möglich ist.

Es besteht zwar grundsätzlich die Möglichkeit, Ultraschall-Transducer vorzusehen, die als Phased-Array ausgeführt sind, und die Abtastung der Schichten auf elektronischem Wege zu bewirken. Auf besonders kostengünstige, konstruktiv einfache und raumsparende Weise kann jedoch ein B-Scan-Applikator mit mehreren Ultraschall-Transducern realisiert werden, wenn er als mechanischer Sektor-Scanner ausgebildet ist, dessen Ultraschall-Transducer, es kann sich hierbei um Einzelwandler oder Wandler-Arrays -mit wahlweise natürlicher, mechanischer oder elektronischer Fokussierung handeln, an einem Rotor angebracht sind, der mittels einer Antriebseinrichtung um eine Achse drehbar ist, wobei die Ultraschall-Transducer bezüglich der Achse im Winkel zueinander versetzt an dem Rotor angebracht sind. Dabei kann vorgesehen sein, daß die Antriebseinrichtung auf der der Fokuszone abgewandten Seite der Stoßwellenquelle hinter dieser angeordnet ist und sich ein Antriebsglied durch eine in der Stoßwellenquelle vorgesehene Bohrung zu dem Rotor erstreckt, so daß eine Verringerung der Apertur der Stoßwellenquelle durch die Antriebseinrichtung vermieden ist.

Gemäß einer Variante der Erfindung weist die Ultraschll-Ortungseinrichtung eine mit dem B-Scan-Applikator zusammenwirkende elektronische Einrichtung auf, mittels derer Ultraschall-B-Bilder der mittels der Ultraschall-Transducer des B-Scan-Applikators abtastbaren Schichten erzeugbar und gleichzeitig darstellbar sind. Das Bedienungspersonal ist so in der Lage, Bewegungen des zu zertrümmernden Konkrementes anhand der gleichzeitig dargestellten Ultraschall-B-Bilder bequem zu erkennen und kann entsprechende Bedienungsmaßnahmen einleiten.

Weitere Ausgestaltungen und Vorteile der Erfindung werden anhand der in den beigefügten Zeichnungen dargestellten Ausführungsbeispiele deutlich. Es zeigen:

Fig. 1 in schematischer Darstellung einen Längsschnitt durch eine erfindungsgemäße Einrichtung,

Fig. 2 eine Ansicht eines Details der Einrichtung nach Fig. 1,

Fig. 3 ein Blockschaltbild der Ultraschall-Ortungseinrichtung der Einrichtung gemäß Fig. 1, und

Fig. 4 eine Ansicht eines Details einer weiteren erfindungsgemäßen Einrichtung.

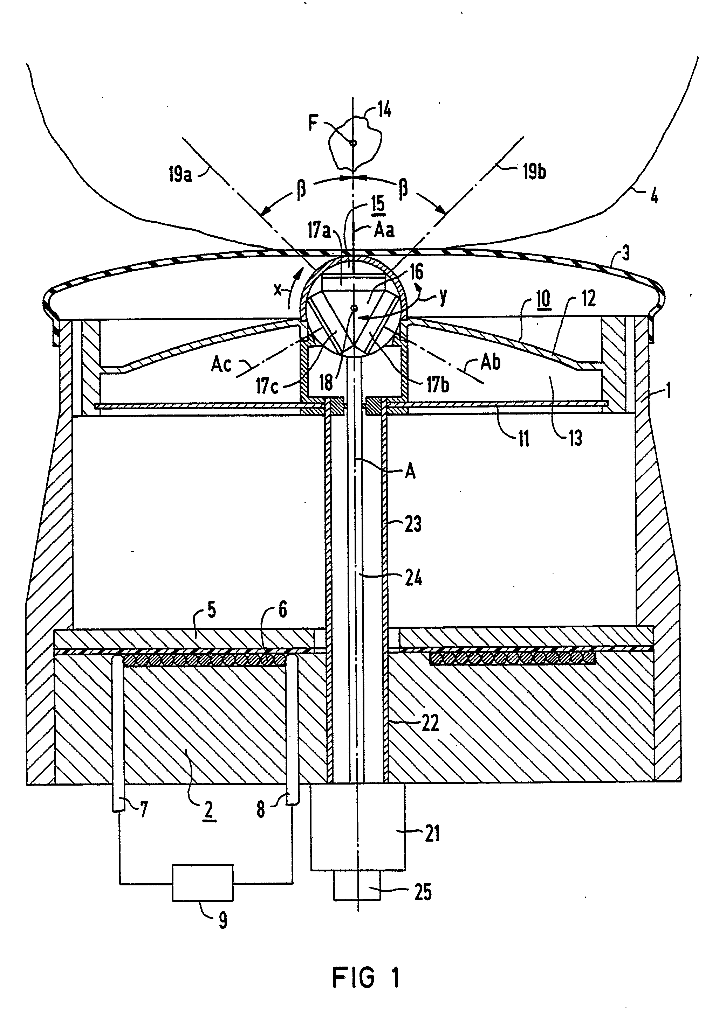

Die in Fig. 1 dargestellte erfindungsgemäße Einrichtung weist ein etwa rohrförmiges Gehäuse 1 auf, an dessen einem Ende ein insgesamt mit 2 bezeichneter Stoßwellengenerator vorgesehen ist. An seinem anderen Ende ist das Gehäuse 1 mittels eines flexiblen Balges 3 verschlossen, der dazu dient, die Einrichtung zur akustischen Koppelung an den im Querschnitt angedeuteten Körper 4 eines zu behandelnden Lebewesens anpressen zu können. Der Innenraum des Gehäuses 1 ist mit Wasser gefüllt, das als akustisches Ausbreitungsmedium vorgesehen ist.

Bei dem Stoßwellengenerator 2 handelt es sich um einen elektro-dynamischen Stoßwellengenerator, wie er in der DE-OS 33 28 051 näher beschrieben ist. Der Stoßwellengenerator 2 weist eine ebene kreisringförmige Membran 5 auf, die mit ihrer einen Seite an das in dem Gehäuse befindliche Wasser grenzt.

Der anderen Seite der aus einem elektrisch leitenden Werkstoff gebildeten Membran 5 gegenüberliegend ist eine Flächenspule 6 mit spiralförmig angeordneten Windungen vorgesehen, die über Anschlüsse 7, 8 mit einer schematisch angedeuteten Generatoreinrichtung 9 verbunden ist, mittels derer sie mit Hochspannungsimpulsen beaufschlagbar ist. Wird die Flächenspule 6 mit einem Hochspannungsimpuls beaufschlagt, bewegt sich die Membran 5 von der Spule 6 schlagartig weg. Infolge dieser Bewegung wird in das Wasser ein im wesentlichen ebener Druckimpuls eingeleitet, der sich auf seinem Weg durch das Wasser zu einer Stoßwelle aufsteilt. Im folgenden wird der Einfachheit halber stets der Begriff Stoßwelle verwendet werden. Die Ausbreitungsrichtung der Stoßwellen entspricht der Richtung der Mittelachse des Stoßwellengenerators 2.

Um die erzeugten ebenen Stoßwellen in der zur Zertrümmerung von Konkrementen erforderlichen Weise fokussieren zu können, ist im Wasser innerhalb des Gehäuses 1 eine insgesamt mit 10 bezeichnete akustische Sammellinse zwischen dem Stoßwellengenerator 2 und dem Balg 3 angeordnet. Die akustische Sammellinse 10 ist als Flüssigkeitslinse ausgeführt. Sie besitzt also eine Eintrittswand 11 und eine Austrittswand 12, zwischen denen eine Linsenflüssigkeit 13 eingeschlossen ist, in der die Schallausbreitungsgeschwindigkeit von der Schallausbreitungsgeschwindigkeit in dem die Sammellinse 10 umgebenden Wasser abweicht. Wenn sich wie im Falle des dargestellten Ausführungsbeispieles in der Sammellinse 10 eine Linsenflüssigkeit 13 befindet, in der die Schallausbreitungsgeschwindigkeit geringer ist als in Wasser, muß die Sammellinse plan-konvex oder bi-konvex ausgebildet sein. Im Falle des in Fig. 1 dargestellten Ausführungsbeispieles ist eine plan-konvexe sphärische Sammellinse 10 vorgesehen. Durchläuft eine ebene Stoßwelle, deren Wellenfront im wesentlichen parallel zur Eintrittswand 11 verläuft, die akustische Sammellinse 10, wird die Stoßwelle auf eine mit F bezeichnete Fokuszone fokussiert. Der Stoßwellengenerator 2 und die Sammellinse 10 bilden also gemeinsam eine Stoßwellenquelle, mittels derer fokussierte Stoßwellen erzeugbar sind, die eine akustische Achse A aufweist, die der Mittelachse des Stoßwellengenerators 2 entspricht und auf der die Fokuskone F der Stoßwellen liegt.

Die Eintrittswand der Sammellinse 10 besteht übrigens aus Polymethylpentene (TPX), während die Ausgangswand aus Teflon (eingetragenes Warenzeichen) besteht. Als Linsenflüssigkeit 13 ist eine Fluor-Kohlenstoff-Flüssigkeit, z.B. Flutec PP3 oder Fluorinert FC 75 (eingetragene Warenzeichen), vorgesehen.

Um die Einrichtung und den Körper 4 des zu behandelnden Lebe wesens relativ zueinander so ausrichten zu können, daß ein im Körper 4 des zu behandelnden Lebewesens befindliches Konkrement 14 sich wie in der Fig. 1 dargestellt im Fokus F der Stoßwellen befindet, ist ein Ultraschall-Sektor-Applikator 15 vorhanden, der als mechanischer Sektor-Scanner ausgebildet ist und es im Zusammenwirken mit einer noch zu beschreibenden elektronischen Einrichtung gestattet, Ultraschall-B-Bilder zu erzeugen. Der Ultraschall-Sektor-Applikator 15 ist in einer im Zentrum der Sammellinse 10 vorgesehenen, flüssigkeitsgefüllten Kammer aufgenommen und befindet sich somit im Sinne der Ausbreitungsrichtung der Stoßwellen vor der Fokuszone F.

Der Ultraschall-Sektor-Applikator 15 enthält eine ungerade Anzahl von Ultraschall-Transducern, nämlich drei in Winkelabständen von 120° an einem Rotor 16 angebrachte Ultraschall-Transducer 17a, 17b, 17c. Der Rotor 16 ist um eine die akustische Achse A rechtwinklig schneidende Achse 18 in Richtung des Pfeiles X drehbar angeordnet, so daß die Ultraschall-Transducer 17a, 17b, 17c aufeinanderfolgend jeweis eine parallel zur Zeichenebene verlaufende sektorförmige Schicht des Körpers 4 des zu behandelnden Lebewesens abtasten. Sämtliche drei sektorförmige Schichten besitzen den gleichen, in Fig. 1 durch strichlierte Linien 19a, 19b angedeuteten Öffnungswinkel, wobei die Linien 19a, 19b mit der akustischen Achse A jeweils den gleichen Winkel "Beta" einschließen. Die Ultraschall-Transducer 17a, 17b, 17c senden, sofern sie entsprechend angetrieben werden, Ultraschallwellen aus, deren Mittelachsen Aa, Ab und Ac durch strichlierte Linien angedeutet sind, wobei in der Darstellung der Fig. 1 die Mittelachse Aa mit der akustischen Achse A zusammenfällt, obwohl sie mit dieser nicht identisch ist.

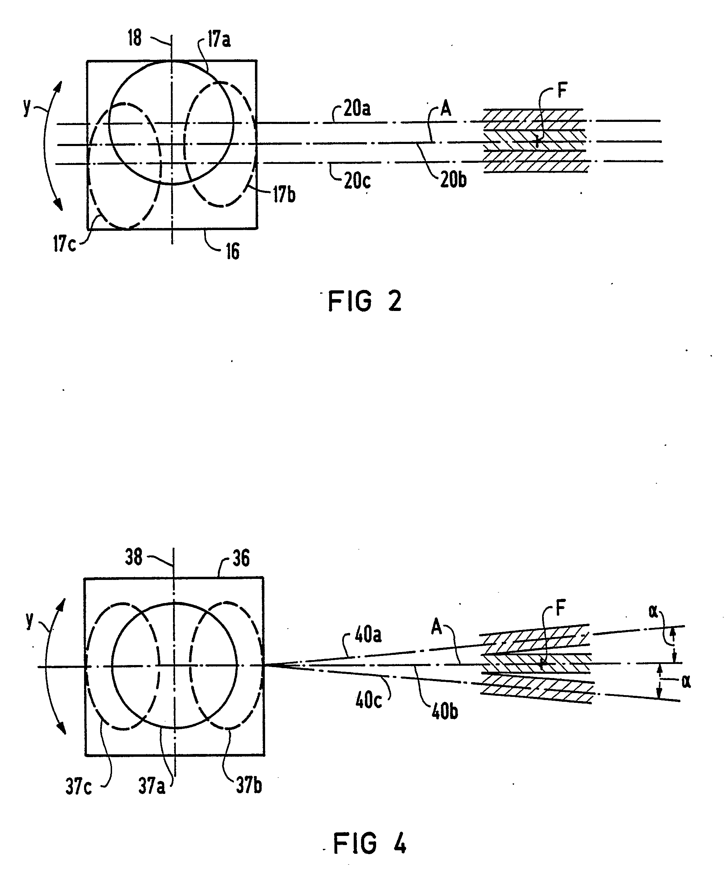

Wie aus der schematischen Darstellung der Fig. 2, in der die Fokuszone F und die akustische Achse A um 90° in die Zeichenebene gedreht dargestellt sind, ersichtlich ist, sind die Ultraschall-Transducer 17a, 17b, 17c an dem Rotor 16 in Richtung der Achse 18 derart seitlich gegeneinander versetzt angeordnet, daß sie sektorförmige Schichten des Körpers 4 des Lebewesens abtasten, deren Mittelebenen 20a, 20b, 20c exakt parallel zueinander und parallel zur akustischen Achse A verlaufen und deren im Bereich der Fokuszone F befindliche Abschnitte, die durch unterschiedliche Schraffur angedeutet sind, einander direkt benachbart sind. Dabei enthält die Mittelebene 20b der mittels des Ultraschall-Transducers 17b abtastbaren mittleren Schicht die akustischen Achse A. Da mittels der Ultraschall-Transducer 17a, 17b, 17c jeweils eine sektorförmige Schicht abtastbar ist, die durch die Linien 19a, 19b gemäß Fig. 1 begrenzt ist, liegen die Winkelhalbierenden der sektorförmigen Schichten mit der akustischen Achse A in einer gemeinsamen Ebene.

Infolge des Umstandes, daß die abtastbaren Schichten den gleichen Öffnungswinkel aufweisen und ihre Winkelhalbierenden in einer gemeinsamen Ebene liegen, ist eine gute Vergleichbarkeit der in den einzelnen Schichten enthaltenen Informationen gewährleistet. Da die Ultraschall-Transducer 17a, 17b, 17c im Sinne der Ausbreitungsrichtung der Stoßwellen vor der Fokuszone F und im Bereich der akustischen Achse A angeordnet sind, können außerdem Informationen über im Ausbreitungsweg der Stoßwellen befindliche Hindernisse, z.B. Rippen, gewonnen werden.

Als Antrieb für den Rotor 16 ist ein Elektromotor 21 vorgesehen, der auf der der Fokuszone F abgewandten Seite der Stoßwellenquelle 2 hinter dieser angeodnet ist. In einer zentralen Bohrung 22 des Stoßwellengenerators ist ein Rohr 23 flüssigkeitsdicht aufgenommen, das sich in Richtung auf die Sammellinse 10 erstreckt und in die den Ultraschall-Sektor-Applikator 15 enthaltende Kammer der akustischen Sammellinse 10 mündet. In der Bohrung des Rohres 23 verläuft eine mittels des Elektromotors 21 angetriebene Welle 24, die über ein nicht dargestelltes Kegelradgetriebe den Rotor 16 antreibt. In dem Rohr 23 verlaufen auch nicht dargestellte elektrische Leitungen, die über ebenfalls nicht dargestellte Schleifringeinrichtungen mit den Ultraschall-Transducern 17a, 17b, 17c verbunden sind und zu der erwähnten elektronischen Einrichtung führen. Die Welle 24 ist übrigens durch einen Dichtring flüssigkeitsdicht in die den Ultraschall-Sektor-Applikator 15 enthaltende Kammer geführt. Mit dem Elektromotor 21 ist ein Signalgeber 25 verbunden, der der jeweiligen Winkelstellung des Rotors 16 entsprechende elektrische Signale abgibt.

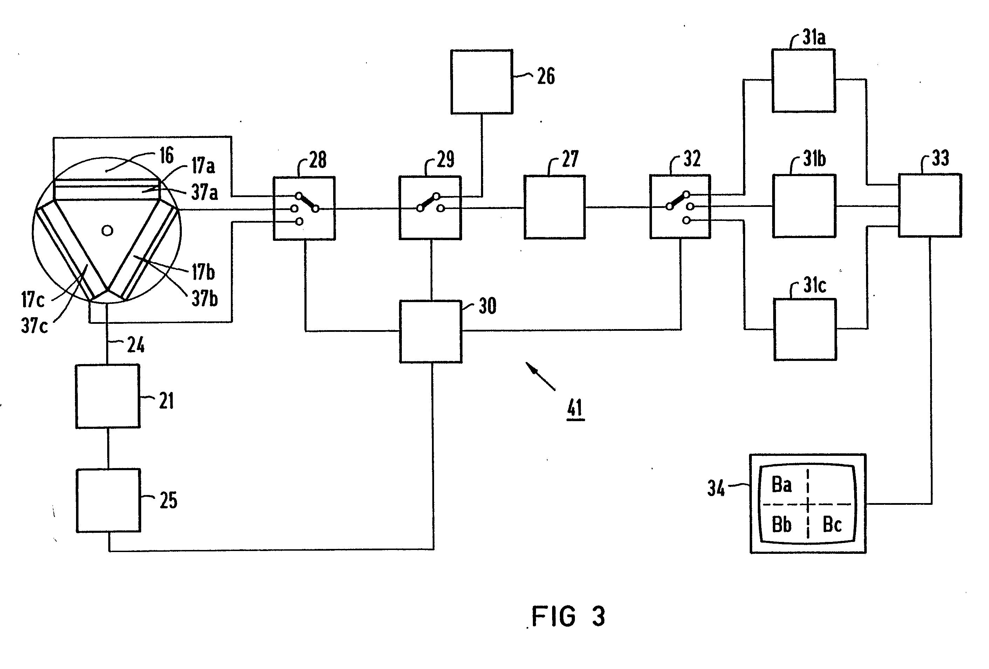

In der Fig. 3 ist die Ultraschall-Ortungseinrichtung der Einrichtung nach den Fig. 1 und 2 als Blockschaltbild dargestellt. Der Ultraschall-Sektor-Applikator 15 mit seinen an dem Rotor 16 angebrachten Ultraschall-Transducern 17a, 17b, 17c, dem Elektromotor 21, der Welle 24 und dem Signalgeber 25 ist schematisch angedeutet. Die Ultraschall-Ortungseinrichtung umfaßt außer dem Ultraschall-Sektor-Applikator 15 eine insgesamt mit 41 bezeichnete elektronische Einrichtung. Diese weist einen an sich bekannten Sender 26, mittels dessen die Ultraschall-Transducer 17a, 17b, 17c des Ultraschall-Sektor-Applikators 15 zur Abgabe von Ultraschall antreibbar sind, und einen ebenfalls an sich bekannten Empfänger 27 für mittels der Ultraschall-Transducer 17a, 17b, 17c empfangene Ultraschall-Echos auf, der die Ultraschall-Echos in Videosignale umwandelt.

Weiter sind erste Schaltmittel 28, mittels derer einer der Ultraschall-Transducer 17a, 17b, 17c anwählbar ist, und zweite Schaltmittel 29 vorhanden, mittels derer der jeweils mittels der ersten Schaltmittel 28 angewählte Ultraschall-Transducer 17a, 17b, 17c wahlweise an den Sender 26 oder den Empfänger 27 anschaltbar ist. Die ersten Schaltmittel 28 sind durch einen 3 zu 1-Analog-Multiplexer gebildet, der in Fig. 3 als dreipoliger Umschalter angedeutet ist. Die zweiten Schaltmittel 29 sind durch einen 1 zu 2-Analog-Demultiplexer gebildet, was in Fig. 3 durch einen zweipoligen Umschalter symbolisiert ist. Mit jedem der Eingänge des Analog-Multiplexers ist einer der Ultraschall-Transducer 17a, 17b, 17c verbunden. Der Ausgang des Analog-Multiplexers ist mit dem Eingang des Analog-Demultiplexers verbunden, dessen einer Ausgang mit dem Sender 26 und dessen anderer Ausgang mit dem Empfänger 27 verbunden ist. Die ersten Schalt mittel 28 und die zweiten Schaltmittel 29, d.h. der Analog-Multiplexer und der Analog-Demultiplexer, werden mittels einer Steuereinrichtung 30 über entsprechende Leitungen in noch zu beschreibender Weise betätigt.

Außerdem ist eine der Anzahl von Ultraschall-Transducern 17a, 17b, 17c entsprechende Anzahl von Video-Speichern 31a, 31b, 31c vorgesehen, von denen wahlweise einer mittels dritter Schaltmittel 32 an den Ausgang des Empfängers 27 anschaltbar ist, wobei der jeweils an den Ausgang des Empfängers angeschaltete Video-Speicher die am Ausgang des Empfängers zur Verfügung stehenden Videosignale unter Überschreibung eventuell gespeicherter Daten speichert. Die dritten Schaltmittel 32 sind durch einen über eine entsprechende Leitung mittels der Steuereinrichtung 30 betätigten 1 zu 3-Demultiplexer gebildet, der in Fig. 3 durch einen dreipoligen Umschalter symbolisiert ist.

Mit den Video-Speichern 31a, 31b, 31c ist eine Bildverarbeitungseinrichtung 33 verbunden, die die in den Video-Speichern 31a, 31b, 31c befindlichen Daten ausliest und derart verarbeitet, daß die mittels der Ultraschall-Transducer 17a, 17b, 17c erzeugten Ultraschall-B-Bilder gleichzeitig auf einem mit der Bildverarbeitungseinrichtung 33 verbundenen Video-Monitor 34 sichtbar sind.

Die Steuerungseinrichtung 30 ist mit dem Signalgeber 25 verbunden, der wie erwähnt der jeweiligen Winkelstellung des Rotors 16 entsprechende Signale abgibt.

Während des Betriebes der erfindungsgemäßen Einrichtung betätigt die Steuerungseinrichtung 30 anhand der Signale des Signalgebers 25 die ersten Schaltmittel 28 derart, daß jeweils derjenige Ultraschall-Transducer 17a, 17b, 17c, der eine solche Lage einnimmt, daß die Mittelachse Aa, Ab, Ac der von ihm ausgehenden Ultraschallwellen sich innerhalb des durch die Linien 19a, 19b begrenzten Sektors befinden, angewählt wird. Gleichzeitig betätigt die Steuerungseinrichtung 30 die dritten Schaltmittel 32 derart, daß der dem jeweils angewählten Ultraschall-Transducer 17a, 17b, 17c entsprechende Video-Speicher 31a, 31b, 31c an den Ausgang des Empfängers 27 angeschaltet ist. Während des Zeitraumes, in dem ein Ultraschall-Transducer 17a, 17b, 17c mittels der ersten Schaltmittel 28 angewählt ist, betätigt die Steuerungseinrichtung 30 die zweiten Schaltmittel 29 rasch aufeinanderfolgend derart, daß der angewählte Ultraschall-Transducer 17a, 17b, 17c abwechselnd an den Sender 26 und den Empfänger 27 angeschaltet ist. Beispielsweise wird der angewählte Ultraschall-Transducer 17a, 17b, 17c während der Abtastung einer sektorförmigen Schicht 256mal abwechselnd an den Sender 26 und den Empfänger 27 angeschaltet. Jedesmal wenn der angewählte Ultraschall-Transducer 17a, 17b, 17c an den Sender 26 angeschaltet ist, betätigt die Steuerungseinrichtung 30 den Sender 26 derart, daß dieser den angewählten Ultraschall-Transducer 27a, 17b, 17c zur Abgabe eines Ultraschall-Impulses antreibt, dessen Echosignale der angewählte Ultraschall-Transducer 17a, 17b, 17c empfängt, der zu diesem Zeitpunkt bereits an den Empfänger 27 angeschaltet ist. Dieser wandelt die Echosignale in zum Aufbau von Ultraschall-B-Bildern geeignete Videosignale um, die über die dritten Schaltmittel 32 dem den jeweils angewählten Ultraschall-Transducer 17a, 17b, 17c entsprechenden Videospeicher 31a, 31b, 31c zur Speicherung zugeführt werden.

Nach einer vollständigen Umdrehung des Rotors 16 befinden sich in den Videospeichern 31a, 31b, 31c Ultraschall-B-Bilder der mittels der Ultraschall-Transducer 17a, 17b, 17c abgetasteten sektorförmigen Schichten des Körpers 4 des zu behandelnden Lebewesens, die in der zuvor beschriebenen Weise unmittelbar aufeinanderfolgend, also quasi-gleichzeitig, gewonnen wurden. Diese Daten liest die Bildverarbeitungseinrichtung 33 aus den Video-Speichern 31a, 31b, 31c aus und stellt die entsprechenden Ultraschall-B-Bilder gleichzeitig auf dem Video-Monitor 34 dar, was durch die Angaben Ba, Bb, Bc angedeutet ist. Jedes der Ultraschall-B-Bilder wird in einem Quadranten des Video-Monitors 34 dargestellt, wobei der verbleibende Quadrant zur Darstellung weiterer Informationen zur Verfügung steht.

Während der nächsten Umdrehung des Rotors 16 wiederholt sich der beschriebene Vorgang, wobei die in den Videospeichern 31a, 31b, 31c befindlichen Daten überschrieben werden. Wird während der gesamten Behandlungsdauer der Betrieb der Ultraschall-Ortungseinrichtung wie beschrieben aufrecht erhalten, können anhand der Darstellung der Ultraschall-B-Bilder Ba, Bb, Bc auf dem Video-Monitor 34 selbst größere Verlagerungen des zu zertrümmernden Konkrementes 14 erkannt werden. Befindet sich das zu zertrümmernde Konkrement 14 wie gewünscht und in Fig. 1 dargestellt in der Fokuszone F der Stoßwellen, befindet es sich in der mittels des Ultraschall-Transducers 17b abtastbaren Ebene und ist auf dem Video-Monitor 34 in dem Ultraschall-B-Bild Bb sichtbar. Tritt einer Verlagerung des zu zertrümmernden Konkrementes 14 quer zur Mittelebene 20b der mittels des Ultraschall-Transducers 17b abtastbaren Schicht um ein solches Maß auf, daß das zu zertrümmernde Konkrement 14 die mittels des Ultraschall-Transducers 17b abtastbare Schicht verläßt, tritt das Konkrement je nach Verschieberichtung entweder in die mittels des Ultraschall-Transducers 17a oder mittels des Ultraschall-Transducers 17c abtastbare Schicht ein und wird demzufolge entweder in dem Ultraschall-B-Bild Ba oder dem Ultraschall-B-Bild Bc sichtbar. Dabei ist die Verlagerung des Konkrementes 14 sofort bei Eintreten der Verlagerung erkennbar, da die Ultraschall-Transducer 17a, 17b die jeweiligen Schichten quasi-gleichzeitig abtasten und die Darstellung der entsprechenden Ultraschall-B-Bilder Ba, Bb, Bc auf dem Video-Monitor 34 gleichzeitig erfolgt. Es besteht also die Möglichkeit, unmittelbar nach Auftreten einer Verlagerung des Konkrementes 14 die Lage der Einrichtung und des Körpers 4 des zu behandelnden Lebewesens relativ zueinander gezielt so zu korrigieren, daß sich das Konkrement wieder in der mittels des Ultraschall-Transducers 17b abtastbaren Schicht befindet. Falls das Konkrement dann eine Lage einnimmt, in der es sich nicht in der Fokuszone F befindet, deren Lage in dem Ultraschall-B-Bild Bb in nicht dargestellter Weise mittels einer Marke, z.B. eines Fadenkreuzes, gekennzeichnet ist, kann in einem weiteren Schritt die Lage der Einrichtung und des Körpers 4 des zu behandelnden Lebewesens so korrigiert werden, daß sich das Konkrement 14 wieder in der Fokuszone F der Stoßwellen befindet, worauf die Behandlung fortgesetzt werden kann.

Der Ultraschall-Sektor-Applikator kann übrigens in nicht näher dargestellter und in Fig. 1 durch den Doppelpfeil Y angedeuteter Weise relativ zur akustischen Achse A verdreht werden, um ihn so ausrichten zu können, daß die Mittelebenen 20a, 20b, 20c der abtastbaren Schichten parallel zur Haupt-Verschieberichtung des Konkrementes 14 verlaufen.

In der Fig. 4 ist in zu der Fig. 2 analoger Weise der Ultraschall-Sektor-Applikator 35 eines weiteren Ausführungsbeispieles dargestellt, das abgesehen von der Ausbildung des Ultraschall-Sektor-Applikators 35 dem zuvor beschriebenen Ausführungsbeispiel entspricht.

Gemäß der Fig. 4 handelt es sich bei dem Ultraschall-Sektor-Applikator 35 wieder um einen mechanischen Sektor-Scanner, der drei in Winkelabständen von 120° an einem Rotor 36 angebrachte Ultraschall-Transducer 37a, 37b, 37c aufweist. Diese sind an dem Rotor 36 derart angeordnet, daß sie sektorförmige Schichten des Körpers 4 des Lebewesens abtasten, deren Mittelebenen 40a, 40b, 40c in Richtung auf die Fokuszone F divergieren, wobei die Divergenz derart gewählt ist, daß die im Bereich der Fokuszone F befindlichen und durch unterschiedliche Schraffur angedeuteten Abschnitte der abtastbaren Schichten einander direkt benachbart sind. Dabei ist die Anordnung derart gewählt, daß die Mittelebene 40b der mittels des Ultraschall-Transducers 37b abtastbaren mittleren Schicht die akustische Achse A enthält. Die Mittelebenen 40a, 40c der mittels der Ultraschall-Transducer 37a, 37c abtastbaren Schichten sind in bezug auf die Mittelebene 40b gegensinnig um einen jeweils gleichen sehr geringen Winkel "Alpha" geneigt, so daß die abtastbaren Schichten bzw. deren Mittelebenen 40a, 40b, 40c näherungsweise parallel zueinander und parallel zur akustischen Achse A verlaufen. Wenn im Zusammenhang mit der Fig. 4 von Mittelebenen 40a, 40b, 40c die Rede ist, ist dies einsichtlich der "Mittelebenen" 40a, 40c nur näherungsweise zutreffend. Genaugenommen handelt es sich hier um kegelmantelförmige Flächen, was jedoch in Anbetracht des geringen Betrages der Winkel "Alpha" vernachlässigbar ist.

Auch die erfindungsgemäße Einrichtung nach Fig. 4 wirkt mit einer elektronischen Einrichtung gemäß Fig. 3 zusammen, was dadurch angedeutet ist, daß in Fig. 3 die Ultraschall-Transducer außer den Bezugsziffern 17a, 17b, 17c zusätzlich mit den jeweils entsprechenden Bezugsziffern 37a, 37b, 37c versehen sind.

**Ansprüche**

1. Einrichtung berührungslosen Zertrümmern von Konkrementen (14) im Körper (4) eines Lebewesens, welche eine Stoßwellenquelle (2, 10) zur Erzeugung von in einer Fokuszone (F) zusammenlaufenden Stoßwellen, eine Ultraschall-Ortungseinrichtung mit einem B-Scan-Applikator (15; 35), mittels dessen zumindest die Fokuszone (F) abtastbar ist, und Mittel (3) zur akustischen Koppelung der Stoßwellenquelle (2, 10) und des B-Scan-Applikators (15; 35) mit dem Körper (4) des Lebewesens aufweist, **dadurch gekennzeichnet,** daß der B-Scan-Applikator (15; 35) mehrere Ultraschall-Transducer (17a, 17b, 17c; 37a, 37b, 37c) enthält, mittels derer wenigstens quasi-gleichzeitig eine der Anzahl der Ultraschall-Transducer (17a, 17b, 17c; 37a, 37b, 37c) entsprechende Anzahl zumindest näherungsweise parallel zueinander verlaufender Schichten abtastbar ist, deren im Bereich der Fokuszone (F) befindliche Abschnitte einander direkt benachbart sind.

2. Einrichtung nach Anspruch 1, **dadurch gekennzeichnet,** daß die Stoßwellenquelle (2, 10) eine akustische Achse (A) aufweist, die durch die Fokuszone (F) verläuft, und daß der B-Scan-Applikator (15; 35) eine ungerade Anzahl von Ultraschall-Transducern (17a, 17b, 17c; 37a, 37b, 37c) enthält, die derart angeordnet sind, daß die mittlere der abtastbaren Schichten die akustische Achse (A) der Stoßwellenquelle (2, 10) enthält.

3. Einrichtung nach Anspruch 1 oder 2, **dadurch gekennzeichnet,** daß die Ultraschall-Transducer (17a, 17b, 17c) relativ zueinander derart angeordnet sind, daß die Mittelebenen (20a, 20b, 20c) der abtastbaren Schichten parallel zueinander verlaufen.

4. Einrichtung nach Anspruch 1 oder 2, **dadurch gekennzeichnet,** daß die Ultraschall-Transducer (37a, 37b, 37c) relativ zueinander derart angeordnet sind, daß die Mittelebenen (40a, 40b, 40c) der abtastbaren Schichten in Richtung auf die Fokuszone (F) divergieren.

5. Einrichtung nach einem der Ansprüche 1 bis 4, **dadurch gekennzeichnet,** daß der B-Scan-Applikator (15; 35) als Sektor-Scanner ausgebildet ist, dessen Ultraschall-Transducer (17a, 17b, 17c; 37a, 37b, 37c) im Sinne der Ausbreitungsrichtung der Stoßwellen vor der Fokuszone (F) im Bereich der akustischen Achse (A) der Stoßwellenquelle (2, 10) angeordnet sind, wobei mittels der Ultraschall-Transducer (17a, 17b, 17c; 37a, 37b, 37c) jeweils eine sektorförmige Schicht abtastbar ist und die Winkelhalbierenden der sektorförmigen Schichten mit der akustischen Achse (A) der Stoßwellenquelle (2, 10) in einer gemeinsamen Ebene liegen.

6. Einrichtung nach einem der Ansprüche 1 bis 5, **dadurch gekennzeichnet,** daß der B-Scan-Applikator (15; 35) als mechanischer Sektor-Scanner ausgebildet ist, dessen Ultraschall-Transducer (17a, 17b, 17c; 37a, 37b, 37c) an einem Rotor (16; 36) angebracht sind, der mittels einer Antriebseinrichtung (21, 24) um eine Achse (18; 38) drehbar ist, wobei die Ultraschall-Transducer (17a, 17b, 17c; 37a, 37b, 37c) bezüglich der Achse (18; 38) im Winkel zueinander versetzt an dem Rotor (16; 36) angebracht sind.

7. Einrichtung nach den Ansprüchen 5 und 6, **dadurch gekennzeichnet,** daß die Antriebseinrichtung (21) auf der der Fokuszone (F) abgewandten Seite der Stoßwellenquelle (2, 10) hinter dieser angeordnet ist und daß sich ein Antriebsglied (24) durch eine in der Stoßwellenquelle (2, 10) vorgesehene Bohrung (22) zu dem Rotor (16; 36) erstreckt.

8. Einrichtung nach einem der Ansprüche 1 bis 7, **dadurch gekennzeichnet,** daß die Ultraschall-Ortungseinrichtung eine mit dem B-Scan-Applikator (15; 35) zusammenwirkende elektronische Einrichtung (41) aufweist, mittels derer Ultraschall-B-Bilder der mittels der Ultraschall-Transducer (17a, 17b, 17c; 37a, 37b, 37c) des B-Scan-Applikators (15; 35) abtastbaren Schichten erzeugbar und gleichzeitig darstellbar sind.

9. Einrichtung nach Anspruch 8, **dadurch gekennzeichnet,** daß die elektronische Einrichtung (41) aufweist:

a) einen Sender (26), mittels dessen die Ultraschall-Transducer (17a, 17b, 17c; 37a, 37b, 37c) des B-Scan-Applikators (15; 35) in der zur Erzielung eines B-Scans erforderlichen Weise zur Abgabe von Ultraschall antreibbar sind,

b) einen Empfänger (27) für mittels der Ultraschall-Transducer (17a, 17b, 17c; 37a, 37b, 37c) empfangene Ultraschall-Echos, der diesen entsprechende, zum Aufbau von Ultraschall-B-Bildern entsprechende Videosignale abgibt,

c) eine der Anzahl der Ultraschall-Transducer (17a, 17b, 17c; 37a, 37b, 37c) entsprechende Anzahl von Video-Speichern (31a, 31b, 31c), von denen je einer einem der Ultraschall-Transducer (17a, 17b, 17c; 37a, 37b, 37c) zugeordnet ist,

d) erste Schaltmittel (28), mittels derer einer der Ultraschall-Transducer (17a, 17b, 17c; 37a, 37b, 37c) anwählbar ist,

e) zweite Schaltmittel (29), mittels derer ein jeweils angewählter Ultraschall-Transducer (17a, 17b, 17c; 37a, 37b, 37c) wahlweise an den Sender (26) oder den Empfänger (27) anschaltbar ist,

f) dritte Schaltmittel (32), mittels derer wahlweise einer der Video-Speicher (31a, 31b, 31c) an den Empfänger (27) anschaltbar ist,

g) eine Steuerungseinrichtung (30), die die ersten Schaltmittel derart betätigt, daß diese zur Abtastung der Schichten periodisch aufeinanderfolgend die einzelnen Ultraschall-Transducer (17a, 17b, 17c; 37a, 37b, 37c) anwählen, die die zweiten Schaltmittel (29) derart betätigt, daß der jeweils angewählte Ultraschall-Transducer (17a, 17b, 17c; 37a, 37b, 37c) während der Abtastung der jeweiligen Schicht abwechselnd zur Aussendung von Ultraschallwellen an den Sender (26) und zum Empfang der entsprechenden Ultraschall-Echos an den Empfänger (27) angeschaltet ist, und die die dritten Schaltmittel (32) derart betätigt, daß der Empfänger (27) an den dem jeweils angewählten Ultraschall-Transducer (17a, 17b, 17c; 37a, 37b, 37c) entsprechenden Video-Speicher (31a, 31b, 31c) angeschaltet ist,

h) einen Video-Monitor (34) zur Darstellung der erzeugten Ultraschall-B-Bilder, und

i) eine mit den Video-Speichern (31a, 31b, 31c) und dem Video-Monitor (34) verbundene Bildverarbeitungseinrichtung (33), die die in den Video-Speichern (31a, 31b, 31c) befindlichen Daten ausliest und derart verarbeitet, daß die Ultraschall-B-Bilder der mittels der Ultraschall-Transducer (17a, 17b, 17c; 37a, 37b, 37c) abgetasteten Schichten gleichzeitig auf dem Video-Monitor (34) sichtbar sind.

10. Einrichtung nach einem der Ansprüche 6 oder 7 und nach Anspruch 9, **dadurch gekennzeichnet,**

daß ein Signalgeber (25) vorgesehen ist, der der Winkelstellung des Rotors (16; 36) entsprechende Signale an die Steuerungseinrichtung (30) gibt, anhand derer die ersten Schaltmittel (28) derart betätigt, daß jeweils derjenige Ultraschall-Transducer (17a, 17b, 17c; 37a, 37b, 37c) angewählt ist, mittels dessen zu einem gegebenen Zeitpunkt die ihm zugeordnete Schicht abtastbar ist.

FIG 1

FIG 2

FIG 4

FIG 3

EP 0 355 175 A1

88 P 3349 E

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 265 742 (SIEMENS) <br> * Spalte 2, Zeile 23 - Spalte 7, Zeile 6; Figuren * | 1,3,6,8 | A 61 B 17/22 <br> A 61 B 8/14 <br> G 01 S 15/89 |
| A | | 5,9 | |
| Y | DE-A-2 719 130 (KRETZTECHNIK) <br> * Das ganze Dokument * | 1,3,6,8 | |
| A | | 9,10 | |
| A | FR-A-2 608 913 (K.K. TOSHIBA) <br> * Das ganze Dokument * | 1-5,8,9 | |
| A,P | EP-A-0 278 303 (SIEMENS) <br> * Zusammenfassung; Figuren * | 1-4 | |
| A | EP-A-0 147 737 (SIEMENS) <br> * Zusammenfassung; Figur * | 1,3 | |
| A | EP-A-0 142 862 (MATSUSHITA) <br> * Seite 3, Zeile 6 - Seite 4, Zeile 1; Zusammenfassung; Figuren 1,2 * | 2,6,7 | |
| A | DE-A-2 722 252 (DORNIER) <br> * Seite 13, Zeile 16 - Seite 14, Zeile 11; Figuren 2,3 * | 4 | RECHERCHIERTE SACHGEBIETE (Int. Cl.4) <br><br> A 61 B <br> G 10 K <br> G 01 S |
| A | EP-A-0 097 917 (SIEMENS) <br> * Zusammenfassung; Figuren * | 6 | |
| A | US-A-4 034 744 (GOLDBERG) | | |
| A | US-A-4 030 344 (NORTHEVED) | | |
| A | US-A-4 328 707 (CLEMENT) | | |

Der vorliegende Recherchenbericht wurde für alle Patentansprüche erstellt

| Recherchenort | Abschlußdatum der Recherche | Prüfer |
|---|---|---|
| DEN HAAG | 19-04-1989 | KLEIN C. |